# EUROPÄISCHE PATENTSCHRIFT

(11) **EP 1 478 389 B1**
(45) Veröffentlichungstag und Bekanntmachung des Hinweises auf die Patenterteilung: **07.01.2009**
(21) Anmeldenummer: 03714635.4
(22) Anmeldetag: 12.02.2003
(51) Int. Cl.: A61K 38/43, A61K 31/404, A61K 31/195, A61P 25/28, A61P 27/02

(54) **THERAPIE VON ERKRANKUNGEN DES AUGES**
TREATMENT FOR DISEASES OF THE EYE
TRAITEMENT DE MALADIES DE L'OEIL

(30) Priorität: 18.02.2002 DE 10206723
(43) Veröffentlichungstag der Anmeldung: 24.11.2004
(73) Patentinhaber: CEVEC Pharmaceuticals GmbH, 51105 Köln (DE)
(72) Erfinder: SCHRAERMEYER, Ulrich, 72076 Tübingen (DE)
(74) Vertreter: Dehmel, Albrecht
(86) Internationale Anmeldenummer: PCT/DE2003/000415
(87) Internationale Veröffentlichungsnummer: WO 2003/070269

(56) Entgegenhaltungen:
- WO-A-00/10507
- WO-A-92/07580
- WO-A-02/066620
- US-A- 5 686 084
- ORLOW SETH J: "Melanosomes are specialized members of the lysosomal lineage of organelles." JOURNAL OF INVESTIGATIVE DERMATOLOGY, Bd. 105, Nr. 1, 1995, Seiten 3-7, XP009012745 ISSN: 0022-202X
- BARRENAS M-L ET AL: "THE INFLUENCE OF EYE COLOUR ON SUSCEPTIBILITY TO TTS IN HUMANS" BRITISH JOURNAL OF AUDIOLOGY, Bd. 25, Nr. 5, 1991, Seiten 303-308, XP009012748 ISSN: 0300-5364 in der Anmeldung erwähnt
- DOREY C K ET AL: "EVIDENCE OF MELANOGENESIS IN PORCINE RETINAL PIGMENT EPITHELIAL CELLS IN-VITRO" EXPERIMENTAL EYE RESEARCH, Bd. 50, Nr. 1, 1990, Seiten 1-10, XP009012760 ISSN: 0014-4835 in der Anmeldung erwähnt
- PETERS S ET AL: "[Characteristics and functions of melanin in retinal pigment epithelium]" DER OPHTHALMOLOGE: ZEITSCHRIFT DER DEUTSCHEN OPHTHALMOLOGISCHEN GESELLSCHAFT. GERMANY DEC 2001, Bd. 98, Nr. 12, Dezember 2001 (2001-12), Seiten 1181-1185, XP002245765 ISSN: 0941-293X
- TATE DAVID J JR ET AL: "Zinc protects against oxidative damage in cultured human retinal pigment epithelial cells." FREE RADICAL BIOLOGY & MEDICINE, Bd. 26, Nr. 5-6, März 1999 (1999-03), Seiten 704-713, XP002245766 ISSN: 0891-5849 in der Anmeldung erwähnt
- SCHRAERMEYER U ET AL: "Up-regulation of tyrosinase in adult mammalian retinal pigment epithelium by phagocytosis of rod outer segments." PIGMENT CELL RESEARCH, Bd. 15, Nr. Supplement 9, 2002, Seiten 32-33, XP009012772 XVIII International Pigment Cell Conference;Egmond aan Zee, Netherlands; September 09-13, 2002, 2002 ISSN: 0893-5785

## Beschreibung

Die vorliegende Erfindung betrifft die Verwendung von Tyrosinase, 5,6-Dihydroxyindol (DHI), 5,6-Dihydroxyindole-2-carboxylsäure (DHICA) und/oder 5-S-Cysteinyldopa. (CD) in Kombination mit Zink zur Herstellung eines Arzneimittels zur Prävention oder Therapie von Erkrankungen des Auges, charakterisiert durch Drusenbildung oder einen erhöhten Gehalt an Lipofuscin.

Tyrosinase ist das wichtigste Enzym zur Synthese von Melanin, ein Pigment welches in Zellen der Haut, des Auges und des Zentralen Nervensystems vorkommt.

Tyrosinase ist ein integrales Membranglycoprotein aus 529 Aminosäuren. Das Enzym katalysiert die Hydroxylierung von Tyrosin zur Dihydroxyphenylalanin (DOPA) und anschließend dessen Überführung in Dopaquinon. Über verschieden Zwischenschritte (Stage-III-Melanosom), die großenteils spontan ablaufen können, entsteht ferner Melanin. Zu diesen Zwischenstufen gehören 5,6-Dihydroxyindol (DHI), 5,6-Dihydroxyindol-2-carboxylsäure (DHICA), und 5-S-Cysteinyldopa (CD).

Nach heutigem Wissensstand findet die Melaninbildung im retinalen Pigmentepithel von Säugetieren inclusive des menschlichen Auges nur pränatal statt ^{2;5}. Beim Menschen ist die Tyrosinase im retinalen Pigmentepithel nur pränatal bis etwa 6 Wochen vor Ende der Schwangerschaft aktiv und wird dann nicht mehr gebildet⁴. Im Auge findet man Melanin bzw. die Tyrosinase in embryonal unterschiedlichen Geweben: in der Uvea, bestehend aus Iris und Chorioidea sowie im Pigmentepithel von Retina (RPE), Iris (IPE) und Ziliarkörper.

Das einzige pigmentiert Gewebe, von dem behauptet wird, dass hier postnatal keine Melaninsynthese mehr stattfindet ist das RPE. Dies geht auf eine Arbeit von Miyamoto und Fitzpatrick (1957) ⁴ zurück, in der gezeigt wurde, dass im postnatalen RPE keine Tyrosinase-Aktivität zu finden ist. Das Enzym selbst wurde nach dem bisherigen Stand der Technik auf Proteinebene in RPE Zellen von adulten Säugetieren oder Menschen nicht nachgewiesen und auch es wurden auch keine weiterführenden Studien unternommen.

Während Melanin in der Haut vorwiegend einen physikalischen Lichtschutz darstellt, sind seine Funktionen im Auge komplexer ⁶. Einen physikalischer Lichtschutz bietet lediglich das Melanin in der Iris (Blendenfunktion). Das Melanin im Retinalen Pigmentepithel (RPE) liegt anatomisch von außen gesehen hinter den zu schützenden sensiblen Photorezeptoren der Netzhaut (NH). Das Pigment kann hier durch Absorption die Streustrahlung reduzieren oder Gewebe, z. B. Endothelzellen der Aderhaut, schützen. Auch hat Melanin chemische Schutzwirkung. Als Radikalfänger reduziert Melanin die Entstehung gewebetoxischer Lipidperoxidationen, die im RPE durch hohe Sauerstoffkonzentrationen, Lichteinfall von außen und ausgeprägte Phagozytose-Aktivität entstehen. Die hohe Sauerstoffkonzentration ist auf eine besonders gute Durchblutung der darunter liegenden Chorioidea zurückzuführen. Die Phagozytose abgeschiedener Außensegmente von Photorezeptoren der Netzhaut ist eine der wesentlichen Aufgaben des RPE und verursacht zusätzlichen oxidativen Stress ¹. Bei der Phagozytose wird extrazellulär und intrazellulär Superoxid frei welches sekundär die Bildung von Hydroxylradikalen und Wasserstoffsuperoxid induziert ¹.

Melanin schützt hier, indem es freie Radikale zu molekularem Sauerstoff und Hydrogenperoxid reduziert. Melanin wirkt daher auch bei Entzündungen des Auges protektiv. Melanin stellt ferner-einen wichtigen Zink-Speicher dar, da es in der Lage ist, Schwermetalle, z.B. Zink, Medikamente und andere zytotoxische Substanzen zu binden und zu speichern Das RPE enthält eine hohe Zink-Konzentration, dessen größter Anteil an Melanin gebunden ist⁷. Da Zink ein notwendiger Cofaktor für ca. 300 Enzyme, z.B. der Superoxiddismutase, der Carboanhydrase, der retinalen Dehydrogenase, der Collagenase und der Katalase⁸ ist, kann ein Verlust von Melanin, z.B. bei AMD, mit einem Funktionsverlust dieser Enzyme einhergehen. Diese zuvor genannte Enzyme haben besonders im Auge wichtige Funktionen. Die Katalaseaktivität ist im Auge von älteren Menschen und besonders bei AMD Patienten reduziert⁹. Dadurch sind die Zellen u.a. weniger vor oxidativen Schäden geschützt. Zink, welches lokal im RPE und in den Melanozyten der Aderhaut durch Verlust von Melanin als auch systemisch im Serum bei AMD erniedrigt ist, kann bestimmte Genexpressionen beeinflussen. Zink wird für zahlreiche u.a. antioxidativ wirkende Enzyme als Co-Faktor benötigt. Ein Mangel bzw. vollständiges Fehlen von Melanin führt z.B. zu folgenden-Erkrankungen des Auges: Albinismus, Hermansky-Pudlak-Syndrom (HPS), Chediak-Higashi-Syndrom (CHS), altersbedingte Makuladegeneration (AMD). Im ZNS korreliert der Morbus Parkinson mit einem Verlust von melaninhaltigen dopaminergen Neuronen der Substantia Nigra. Der Melaningehalt des Innenohres korreliert mit der Empfindlichkeit gegen oxidativen Stress verursacht durch Lärm¹⁰.

Eine kausale Therapie für Albinismus, Hermansky-Pudlak-Syndrom und Chediak-Higashi-Syndrom ist bisher nicht bekannt. Bei Albinos wird der extremen Photophobie durch Verwendung von Sonnenbrillen entgegengewirkt.

Eine kausale Therapie der AMD ist derzeit nicht bekannt. Behandlungen durch Argonlaserkoagulation könnten nur bei nicht subfovealen CNV angewendet werden und sie sind ebenso wie die Photodynamische Therapie nicht in der Lage, Rezidive zu verhindert.

Die derzeit eingesetzten Standardtherapien, bei Morbus Parkinson mit L-DOPA zeigen Nebenwirkungen und verlieren nach einigen Jahren oft ihre Wirksamkeit. Auch Dopaminagonisten, MAO-Hemmer, Amantadin und Antocholinergica zeigen nebenwirkungen oder beeinflussen nur einzelne Auswirkungen der Erkrankung.

Der vorliegenden Erfindung liegt daher die Aufgabe zugrunde, Mittel zur Prävention oder Therapie von Erkrankungen bereitzustellen, die durch Drusenbildung oder einen erhöhten Gehalt an Lipofuscin charakterisiert sind.

Die Aufgabe wird durch den in den Patentansprüchen definierten Gegenstand gelöst.

Die Erfindung wird durch die nachfolgenden Figuren näher erläutert:

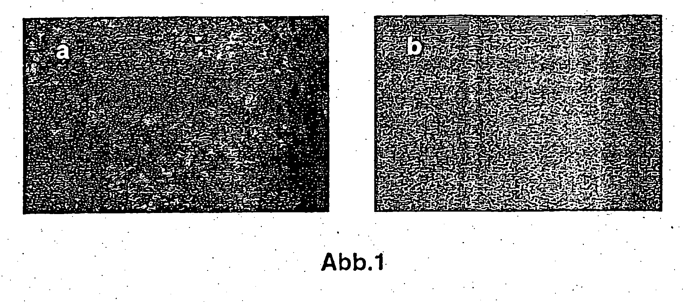

Figur 1A zeigt humane RPE Zellen bei denen die Tyrosinase durch PTU gehemmt wurde nach 15 Fütterungen mit Sehzellmembranen. Die Zellen, zeigen eine intensive, goldgelbe Autofluoreszenz, die für Lipofuszin spezifisch ist.
In Figur 1B ist schematisch gezeigt, dass die Entstehung von Lipofuszin und die damit verbundenen Autofluoreszenz in Zellen kaum vorhanden war, die ansonsten genauso behandelt wurden wie die in Figur 1A gezeigten Zellen, wenn die Tyrosinase nicht durch PTU gehemmt war.

Der hier verwendete Begriff "Tyrosinase" bezeichnet ein Protein oder Polypeptid mit der Aminosäuresequenz spezifisch für ein Enzym mit der biologischen Aktivität der Tyrosinase wie vorstehend beschrieben. Ein Beispiel für eine Tyrosinase ist die humane melanogenetische Tyrosinase (EC 1.14.18.1). Diese Tyrosinase ist ein integrales Membranglycoprotein aus 529 Aminosäuren (GenBank-Nummer M27160). Die Tyrosinase gehört zusammen mit den humanen Tyrosinase-Related Proteinen I+II (TRP I+II) (GenBank-Nummer für TRP I ist AL138753 und für TRP II D17547), Lysosome Associated Membrane Protein (Lamp; GenBank-Nummer P11279) und gp100 (pmel 17) (GenBank-Nummer P40967) zu einer Proteinfamilie. Der Begriff Tyrosinase schließt daher die zu der Proteinfamilie zählenden Proteine mit ein sowie eventuelle Modifikationen der Tyrosinase z.B. posttranslationale Modifikationen.

Ferner umfaßt der Begriff Tyrosinase eine Nukleinsäure mit der Sequenz kodierend für eines der vorstehend erläuterten Polypeptide. Dabei fallen nicht nur die bisher bekannten Sequenzen unter den Begriff sondern auch die Sequenzen die Derivate, d.h. Substitutionen, Additionen, Deletionen, Insertionen, Inversionen umfassen und auch die Sequenzen, die Fragmente und Modifikationen aufweisen. Die Nukleinsäure umfasst nicht nur die jeweilige cDNA, sondern kann ferner den genomischen Locus einschließlich der Introns, Regulationselemente etc. entfalten. Eine Nukleinsäure fällt unter den Begriff Tyrosinase, wenn das von ihr codierte Protein oder Polypeptid die biologische Aktivität der Tyrosinase aufweist.

Der hier verwendete Begriff "Vektor" oder "Gentransfervektor" bezeichnet natürlich vorkommende oder künstlich erschaffene Konstrukte und Organismen zur Aufnahme, Vermehrung, Expression oder Übertragung von Nukleinsäuren in Zellen. Beispiele für Gentransfervektoren sind Viren, wie Adenoviren, Adeno-assoziierte Viren, Lentiviren, Retroviren, Pockenviren, Alphaviren, Bakuloviren, Tollwutviren oder Herpesviren. Die Gentransfervektoren besitzen die Fähigkeit, sich in einer Zelle autonom zu vermehren oder ins Genom der Zellen zu integrieren. Der Gentransfervektor ist so aufgebaut, dass er mindestens ein gewünschtes therapeutisches Polynukleotid in der Art enthält, dass dieses repliziert und/oder exprimiert wird. Ferner können die Gentransfervektoren weitere Polynukleotide wie einen Selektionsmarker enthalten.

Oxidativer Stress wird z.B. durch Phagozytose ausgelöst. Überraschenderweise fand der Erfinder, dass die Tyrosinase und deren Metabolite wie z.B. 5,6-Dihydroxyindol (DHI), 5,6-Dihydroxyindole-2-carboxylsäure (DHICA) und/oder 5-S-Cysteinyldopa (CD) Schutzfunktion zu Zeitpunkten erhöhter Belastung durch reaktive Sauerstoffspezies bieten könnten und Zellen und Gewebe vor oxidativem Stress schützen.

Unter oxidativem Stress versteht man die Entstehung von reaktiven Sauerstoffspecies in Zellen oder Geweben. Diese Sauerstoffspecies sind Superoxidanion, Hydroxylradikal und H₂O₂. Diese Species entstehen zum Beispiel bei den Reaktionen der Atmungskette in den Mitochondrien jeder Zelle. Das Risiko einer Zellschädigung steigt, wenn das Gewebe hohe Soffwechselaktivität hat (z.B. Gehirn) oder stark lichtexponiert ist (Auge, Haut). Die Sauerstoffspecies (Superoxidanion, H₂O₂) werden durch antioxidative Enzyme (Superoxiddismutase, Katalse Glutathionperoxidase und andere) eliminiert. Gelingt diese Elimination jedoch nicht vollständig, entsteht Hydroxylradikal, welches schwere Zellschäden und Zelltod verursacht. Zellalterung und Alterung ganzer Organismen werden auf wie Schäden zurückgeführt, die durch reaktive Sauerstoffspecies verursacht werden. Ein sehr bekannter Zellschaden durch oxidativen Stress ausgelöst ist die Lipidperoxidation. Oxidativer Stress verursacht neurologische degenerative Erkrankungen (z.B. Morbus Parkinson, Altersbedingte. Makuladegeneration, Alzheimer, degenerative Erkrankungen des Innenohres) und Herzkreislauferkrankungen (z.B. Herzinfarkt; Artherosklerose) aber auch Tumorentstehung (z.B. Melanom).

Lipofuszin ist ein braun-gelbes, elektronendichtes, autofluoreszeierendes Pigment, das in den Lysosomen postmitotischer, Zellen z.B. Nervenzellen, Herzmuskelzellen, Pigmentepithelzellen des Auges und Hautzellen, insbesondere wenn sie oxidativem Stress ausgesetzt sind, akkumuliert. Oxidativer Stress verursacht Lipidperoxidation. Diese peroxidierten Lipide lassen sich nicht mehr vollständig abbauen und werden teilweise in Lipofuszin überführt. Lipofuszin ist besonders im retinalen Pigmentepithel von erheblicher pathologischer Bedeutung und wird vermutlich durch unvollständigen Abbau von Sehzellaußengliedern verursacht. Diese werden wegen ihres hohen Gehaltes an ungesättigten Fettsäuren besonders leicht in Lipofuszin überführt, wenn reaktive Sauerstoffspecies vorhanden sind. Durch den Gehalt an Lipofuszin kann auf das Alter einer Zelle geschlossen werden. Die Lipofuszinflecken in der Haut alternder Menschen werden als Alterspigment bezeichnet. Die genaue chemische Zusammensetzung des Lipofuzins ist nicht bekannt. Eine Komponente besteht aus Ethanolamin.

Drusen sind Ablagerungen von zellulärem Restmaterial unbekannter Herkunft unter dem Pigmentblatt, zwischen oder innerhalb der Bruch'schen Membran und dem RPE. Es ist möglich, dass sie als Folge von Phagozytose-und Abbaustörung retinaler Pigmentzellen entstehen; und es ist sicher, dass bei komplexen Erkrankungen des Retina-, Pigmentepithel-, Aderhautkomplexes, einschließlich AMD beim Menschen, Drusen eine Rolle spielen. Funduskopisch unterscheidet man sogenannte weiche Drusen mit unscharfer Begrenzung oder harte Drusen mit scharfen Grenzen. Entstehen Drusen im Bereich der Makula, ist dieses mit starkem Visusverlust verbunden.

Die vorliegende Erfindung besteht in der Verwendung von Tyrosinase, 5,6-Dihydroxyindol (DHI), 5,6-Dihydroxyindole-2-carboxylsäure (DHICA) und/oder 5-S-Cysteinyldopa (CD) in Kombination mit Zink zur Herstellung eines Arzneimittels zur Behandlung oder Prophylaxe von Erkrankungen, die durch oxidativen Stress hervorgerufen werden. Insbesondere werden die Arzneimitte bei Augenerkrankungen zur Behandlung von Drusen oder eines erhöhten Gehalts an Lipofuszin oder zur Prophylaxe der Drusenbildung oder Lipofuszinbildung verwendet.

Dabei kann Zink gleichzeitig oder nach den anderen in der Kombinationszusammensetzung verwendeten Substanzen verabreicht werden. Vorzugsweise wird zuerst die Konzentration an Melanin in den von den Erkrankungen betroffenen Zellen, z.B. der Aderhaut, der Substantia Nigra oder im RPE, mittels Verabreichung der vorstehend beschriebenen Gentransfervektoren erhöht. Anschließend wird dann die Konzentration von Zink in den künstlich pigmentierten Geweben durch Substitution mit Zink erhöht, wodurch dann die antioxidativen Schutzeffekte des Melanins und seiner Metaboliten verbessert werden". Die Verabreichung von Zink kann auf oralem Weg erfolgen. Die Blutgefäße der Aderhaut und Neuronale Zellen im ZNS können geschützt werden, indem oxidativer Stress reduziert wird. Die Neubildung von Blutgefäßen, die Entstehung von Lipofuzin und Drusen kann durch die erfindungsgemäße Kombinationsbehandlung reduziert werden.

Die nachfolgenden Beispiele erläutern die Erfindung.

### Beispiel 1: Induktion von Tyrosinase durch Phagozytose in vitro

Humane retinale Pigmentepithelzellen wurden 2 Stunden post mortem aus Organspenderaugen isoliert, in dem sie in 0,2 % Trypsinlösung mit 0,5 mM EDTA (soweit nicht anders angegeben stammten die Reagenzien von Sigma, Deisenhofen, Germany) 30 min inkubiert wurden. Die Zellen wurden zentrifugiert und in 24 Well plates (Nunc, Mainz, Germany) in Dulbeccos modified Eagle Medium mit 15 % fötalem Kälberserum, 50 µg/ml Gentamycin und 2,5 µg/ml Amphotericin kultiviert. Die Zellen blieben 3 Wochen bis zur Konfluenz in Primärkultur (P0). Nachfolgende Passagierung erfolgte durch Trypsinierung der Zellen, in den entsprechenden Zeitintervallen. Für die Fütterung mit Sehzellmembranen wurden Zellen von 3 verschiedenen. Spendern (Passage 3,6 und 16 benutzt).

### Beispiel 2: Isolation von Sehzellmembranen

Isolierte Netzhäute aus Rinderaugen wurden 2 min in KCL-Puffer (0.3 M KCI, 10 mM Hepes, 0.5 mM CaCl₂, 1mM MgCl₂ and 48% sucrose) bei pH 7 kräftig geschüttelt und dann bei 2000 rpm in einer Tischzentrifuge (Type ZJ 1, Christ Germany) für 5 min zentrifugiert. Der Überstand wurde filtriert durch einen Mullfingerling und verdünnt mit KCI-Puffer (1:1) und nochmals zentrifugiert bei 2500 rpm für 10 min. Die isolierten Stäbchenaußensegmente wurden zweimal durch Zentrifugation in 10 mM Tris-Puffer (Trizma Base) gewaschen bei pH 4, pelletiert und bis zur Verwendung bei minus 80 Grad eingefroren.

### Beispiel 3: Phagozytose von Sehzellmembranen

RPE Zellen, die 2 Wochen an die Kulturbedingungen adaptiert waren, wurden mit ca. 2 x 10⁶ Stäbchenaußensegmenten überschichtet. Nach 4 Stunde wurden die nacht phagozytierten Sehzellmembranen ausgewaschen und durch frisches Medium ersetzt. Nach 0, 5, 20 und 40 Stunden wurden die Zellen in 4% Paraformaldehyd fixiert oder in Trireagent aufgelöst und für die verschiedenen Auswertungen verwendet. Als Kontrollen dienten ungefütterte Zellen.

### Beispiel 4: Histochemischer Nachweis von Tyrosinase mit DOPA

Ein Teil der Zellen wurden ungefüttert bzw. 20 Stunden nach Inkubation mit Sehzellmembranen in 2 % Glutaraldehyd in 100 mM Natriumkakodylatpuffer 2 Stunden fixiert (pH 6,8) und mit 5 mM L-Dihydroxyphenylalanin (L-DOPA) 1 Stunde inkubiert. Anschließend wurden die Zellen nach Routinemethode für die Elektronenmikroskopie eingebettet, ultradünngeschnitten und in einem Zeiss EM 9 (Oberkochem, Germany) Elektronenmikroskop ausgewertet.

In gefütterten Zellen waren elektronendichte Golgiapparate und zahlreiche cytoplamatische Vesikel vorhanden. Gelegentlich waren Phagosomen nachweisbar, die als Folge der DOPA Reaktion elektronendicht geworden waren. In ungefütterten Zellen wurde elektronendichtes Material als Folge der DOPA Reaktion nicht gefunden.

### Beispiel 5: Nachweis von Tyrosinase durch Immunhistochemie an kultivierten Zellen

Auf Objektträgern kultivierte Zellen wurden fixiert in 4 % Paraformaldehyd in 0,1 M PBS für 15 min., gewaschen in 0.05 M TBS (Trishydroxymethylaminomethane), permeabilisiert in 0,25 % Triton (Serva) und blockiert mit 5 % BSA (Bovine Serum Albumine) für 40 min.. Die Zellen wurden mit dem 1. anti-Tyrosinase monoklonalen Antikörper (Chemicon International Inc.) (1:150) über Nacht inkubiert. Die Inkubation mit dem sekundären Cy2 konjugierten Antikörper (AffiniPure Goat Anti-Mouse IgG, Dianova) (1:100) dauerte 1 Stunde. In einem weiteren Schritt wurden die Präparate mit anti-Rhodopsin Antikörpern (Leinco. Techn Instr., verdünnt 1:50) über Nacht inkubiert. Die primären Antikörper wurden mit anti-Maus IgG gekoppelt an den Fluoreszensfarbstoff Cy3 (Rockland, Gilbertsville, PA, USA) sichtbar gemacht. Die Zellen wurden unter dem Fluoreszenzmikroskop (Axiophot, Zeiss, Oberkochem, Deutschland) ausgewertet. Es jeweils 6 Gesichtfelder mit ca. 50 Zellen analysiert.

| | Zellen mit Tyrosinase enthaltenden Vesikeln in % | | Zellen mit Tyrosinase und Rhodopsin enthaltenden Vesikeln in % | |
|---|---|---|---|---|
| 24 Stunden nach Inkubation mit ROS | 8 ± 10,2 | n = 6 | 0,5±0,6 | n = 6 |
| Ohne ROS | 1 ±1,3 | n=6 | 0±0 | n=6 |
| student's t-test | p = 0,00019 | | | |

### Beispiel 6: Nachweis von Tyrosinase in retinalen Pigmentepithelzellen von Ratten

Augen von Long Evans Ratten, die unter einem 12 Stunden hell-dunkel Lichtzyklus gehalten wurden (Licht an um 7 Uhr morgens), wurden um 8 Uhr und 10 Uhr entnommen. Die Augen' wurden enukleiert, in Höhe des Limbus mit einem Scalpel angestochen und über Nacht in 4 % Paraformaldehyd fixiert. Am nächsten Tag wurde der Vorderabschnitt der Augen bis kurz hinter die Ora serrata durch einen umlaufenden Schnitt abgetrennt. Durch 4 radiale Schnitte wurde der verbleibende Augenbecher in Quadranten geteilt und die Netzhäute wurden entfernt. Quadranten, bestehend aus Pigmentepithel, Chorioidea und Sklera, die Lasemarben enthielten, wurden 4 x 10 min in Tris-Puffer (TBS) und dann 10 min in 0,5 M NH₄CL und 0,25 % Triton (Serva, Heidelberg, Deutschland) inkubiert. Nach zwei weiteren Spülungen wurden die Präparate mit 5 % BSA (Albumin, Bovine Fraktion) inkubiert. Die Flatmounts wurden mit Anti-Rhodopsin- und anti-Tyrosinase Antikörper inkubiert, wie für die kultivierten Zellen beschrieben wurde. Zur Kontrolle wurden die Zellen mit monoklonalen Antikörpern gegen Tyrosinhydroxylase aus Ratten- Phäochromocytom (Boehringer, Mannheim, Germany) 1:500 verdünnt inkubiert. Die "Flatmount-Präparate" wurden unter dem Fluoreszenzmikroskop (Axiophot, Zeiss, Oberkochem, Deutschland) ausgewertet. Die Präparate wurden mit den entsprechenden Filtersets für Cy2 und Cy3 betrennt mit einer digitalen Kamera photographiert und mit Openlab Software (Improvision, UK) überlagert Zellen, die Tyrosinase positive-, Tyrosinase und Rhodopsin positive Vesikel enthielten wurden gezählt. Alle Zellen waren negativ für Tyrosinhydroxylase. Die Anzahl der positiven Zellen ist in der nachfolgenden Tabelle dargestellt. Es wurden jeweils Gesichtfelder mit im Mittel 250 Zellen ausgewertet.

| | Zellen mit Tyrosinase enthaltenden Vesikeln in % | | Zellen mit Tyrosinase, und Rhodopsin enthaltenden Vesikeln in % | |
|---|---|---|---|---|
| 8 Uhr | 0,6 ± 0,3 | n = 23 | 0,3 ± 0,3 | n = 11 |
| 10 Uhr | 1,2 ± 3,3 | n = 30 | 0,2 ± 0,4 | n=16 |
| student's t-test | p = 0,01 | | | |

### Beispiel 7: Hemmung der Tyrosinase verstärkt die Bildung von Lipofuszin in RPE Zellen

Humane RPE Zellen, die 2 Wochen an die Kulturbedingungen adaptiert waren, wurden in 3 Versuchsgruppen eingeteilt.
1.) Die Zellen bekamen 1 mM Phenylthioharnstoff (Phenylthiourea PTU, Sigma, Deisenhofen, Germany), einen Inhibitor der Tyrosinase ins Kulturmedium
2.) Die Zellen in Gruppe 2 wurden zur Kontrolle ohne PTU kultiviert
3.) Die Zellen in Gruppe 3 wurden wie in Gruppe 1 mit PTU kultiviert aber nicht gefüttert.

Die Zellen in Gruppe 1+2 wurden mit ca. 2 x 10⁶ Stäbchenaußensegmenten überschichtet. Die Konzentration von BTU blieb stets konstant. Nach 4 Stunden wurden die nicht phagozytierten Sehzellmembranen ausgewaschen und durch frisches Medium ersetzt. Dieses geschah an 15 aufeinanderfolgenden Tagen. Danach wurden die Zellen unter dem Floureszensmikroskop (Axiovert, Zeiss Oberkochem, Germany) mit Filtern angeschaut, die für die Autofluoreszens des Lipofuszins spezifisch sind (Anregung 360 nm, Emission 540 nm, Feuerbacher Analysentechnik, Tübingen, Germany) und wurden mit einer digitalen Kamera photographiert und mit Openlab Software (Improvision, UK) gespeichert.

RPE Zellen der Gruppe 1 bei denen die Tyrosinase durch PTU gehemmt wurde, zeigten nach der 15 tägigen Fütterung mit Sehzellmembranen intensive, goldgelbe Autofluoreszens, die für Lipofuszin spezifisch ist (Abb.1a). Diese Autofluoreszens war in Zellen der Gruppe 2 nur schwach ausgeprägt (Abb.1b). Zellen, die nur mit PTU inkubiert wurden (Gruppe 3) zeigen keine Autofluoreszens. Tyrosinase verhindern die Entstehung von Lipofuszin im retinalen Pigmentepithel.

### Beispiel 8: Western Blot

Western Blot wurde mit kultivierten humanen RPE-Zellen vor und 5 und 40 Stunden nach Fütterung mit Sekzellmembranen durchgeführt. Zwei Melanomzellinien wurden zur Kontrolle der Tyrosinaseexpression mitverwendet. Die Proteinproben (je 75 µg) wurden entweder auf seine NC-membrane (M1) oder eine PVDF-membrane (polyvinylidine difluoride membrane, M2) aufgetragen. M1 wurde gewaschen in TBS, mit India Ink gefärbt und nochmals gewaschen. M2 wurde mit Aqua dest. gewaschen, mit PonceauS gefärbt und nochmals gewaschen. Die Reaktion wurde gestoppt mit 5% BLOT- Quick Blocker (Chemicon) und TBS-T (0,05 % Tween-20, 1xTBS) für 1 Stunde bei Raumtemperatur. Die Proben wurden mit 20 ml Maus anti-Tyrosinase ' monoklonalem Antikörper (DUNN/Chemicon MS 800) 1:500 verdünnt in TBS-T bei 4°C über Nacht inkubiert Nach Waschen mit 20 ml TBS wurde der sekundare Antikörper HRP-anti-mouse IgG (Horse radish peroxidase, Verdünnung 1:6000 in TBS-T) 2 Stunden bei Raumtemperatur inkubiert und dann ausgewaschen. Alle Proben wurden mit monoklonalen Antikörpern gegen humanes ß-Aktin (Sigma, Deisenhofen, Germany) 1:800 verdünnt über Nacht inkubiert. Peroxidase wurde sichtbar gemacht mit LumiLight 1:1 Vol. Luminol/Enhancer. Die Peroxidaselösung wurde in einem Plastikfilm inkubiert.

Eine stark postive Bande mit Molekulargewicht von 75 KD zeigte sich in den Melanomzellen. Die gleiche Bande war in ungefütterten RPE Zellen sehr schwach nachweisbar und verdoppelte sich in der Dicke 5 Stunden nach Fütterung, um 40 Stunden nach Fütterung auf ihren Ausgangswert vor der Filterung zurückzukehren. Die Intensität der Banden für β-Actin blieb in allen Proben konstant.

Die Ergebnisse zeigen eine Zunahme der Tyrosinaseexpression auf Proteinebene als Folge der Inkubation mit Sehzellmembranen.

### Beispiel 9: Erhöhung der intrazellulären Zinkkonzentration in pigmentiertem Gewebe

Zwei Albino (Wistar) Ratten und 2 pigmentierte (Long Evans) Ratten wurden intraperitoneal mit 40 mg/Kg Körpergewicht ZnCl₂ injiziert. Nach 24 Stunden wurde der Chorioidea-RPE-Komplex von Retina und Sklera isoliert. Entsprechende Proben von unbehandelten Tieren dienten als Kontrollen. Die Proben wurden direkt in dicht schließende PFA-vessels (Savillex) eingewogen (Microbalance Sartorius MC21S) und mittels Ultraschallhomogenisierung für 24h bei 30°C mit HNO₃-H₂0₂ (suprapur grade reagents; Merck) aufgeschlossen. Nach vorsichtiger Evaporation wurden die Reste in 2 % HNO₃ für 5 h bei 50°C gelöst. Diese Lösung wurde dann in einen 10 ml volumetrischen Kolben überführt. Die entgültige klare Lösung enthielt 10 ng Rhodamin (Rh)/ml⁻¹ und wurde mit HNO₃ 2% aufgefüllt. Der Nachweis von Zink wurde mit induktiv gekoppelter Plasmamassenspektrometrie (ICP-MS, ELAN 6000 Perkin-Elmer/Sciex) und unter Verwendung von Rh als internem Standard und einer Kalibrierung mit chemischen Reagentien des höchsten Reinheitsgrades durchgeführt.

Die Zinkkonzentration des Chorioidea-RPE Komplexes betrug bei unbehandelten Albinoratten 5,1 µg/g, bei pigmentierten Ratten 74 µg/g und stieg im Gewebe von Albinos nicht an (6,5ug/g), erhöhte sich jedoch um den Faktor 1.6 in pigmentierten Ratten (125 µg/g) 24 Stunden nach Injektion von Zink. In pigmentierten Ratten war die Endkonzentration an Zink nach Injektion 19 mal höher als in den entsprechend behandelten Albinos.

### Beispiel 10: Erhöhung der intrazellulären Zinkkonzentration und weiter Elemente in künstlich pigmentiertem Gewebe

In 8 Augen von Albino (Wistar) Ratten wurde subretinal 0,008 mg Melanin von Tintenfischen (Sepia officinalis, Sigma) gelöst in 1 µl physiologischer Salzlösung injiziert. Nach 3 Tagen wurden die Tiere intraperitoneal mit 40 mg/Kg Körpergewicht ZnCl₂ injiziert. Entsprechende Proben von unbehandelten Albinos dienten als Kontrollen (4 Augen). Nach 24 Stunden wurden die künstlich pigmentierten Chorioidea-RPE-Komplexe isoliert von Retina and Sklera. Die Proben wurden direkt eingewogen (Microbalance Sartorius MC21S) in 7 ml dicht schließende PFA-vessels. (Savillex) und aufgeschlossen (24h/30°C mit Ultraschallhomogenisierung) mit HNO₃-H₂O₂ (suprapur grade reagents (Merck)). Der Nachweis von Zink wurde mit induktiv gekoppelter Plasmamassenspektrometrie durchgeführt wie zuvor beschrieben.

Die Zinkkonzentration des Chorioidea-RPE Komplexes war in künstlich pigmentierten Geweben 49,6 µg/g und ohne Melaninijektion 5,1 µg/g. Das entspricht einer Erhöhung der Zinkkonzentration um den Faktor 9,7 im Vergleich zu Gewebeproben von unbehandelten Albinoratten erhöht 24 Stunden nach Injektion von Zink. Auch die Konzentration der Elemente Ca, Mn, Fe, Co, Ni, Cu und Cd wurde durch Injektion von Melanin deutlich erhöht in Richtung der Werte, die bei pigmentierten Tieren normal sind (siehe nachstehende Tabelle).

**Tabelle: Konzentrationen in µg/g Nassgewicht RPE-Chorioidea-Komplex**

| | Ca | Mn | Fe | Co | Ni | Cu | Zn | Cd |
|---|---|---|---|---|---|---|---|---|
| Albino-Ratte | 37 | 0,11 | 7,7 | 0,01 | 0,33 | 0,77 | 5,1 | 0,01 |
| Albino Ratte + Melanininjektion | 634 | 0,65 | 97 | 0,08 | 1 | 5,08 | 49,6 | 0,02 |
| pigmentierte Ratte | 2823 | 1,56 | 94 | 0,13 | 4 | 14,1 | 74 | 0,07 |

Diese Ergebnisse zeigen, dass durch eine künstliche Erhöhung der Melaninkonzentration die . Konzentration an Spurenelementen im Gewebe erhöht werden kann. Diese Erhöhung kann einerseits die physiologischen Funktionen der Zellen unterstützen, andererseits können auch toxische Elemente (Fe, Cd) vom Melanin fest gebunden werden, so dass das Zytopläsma der Zellen vor den schädlichen Auswirkungen dieser Elemente geschützt wird.

### Beispiel 11: Erhöhung der mRNA für Katalase durch Injektion von Zink

Albino (Wistar) Ratten und pigmentierte (Long Evans) Ratten- wurden intraperitoneal mit 40 mg/Kg Körpergewicht ZnCl₂ injiziert. Nach 6 und 24 Stunden, wurde der Chorioidea-RPE-Komplex isoliert von Retina and Sklera. Die Menge an Katalase mRNA Expression in Chorioidea-RPE-Komplex wurde mit Real Time RT-PCR mit dem SYBR Green I Reactionssystem in einem iCycler ( (Bio-Rad Laboratories, Hercules, CA) quantifiziert. PCR-Primer (Katalase: upper primer: TAG CCA GAA GAG AAA CCC ACA AAC T (SEQ ID NO:1) und lower primer: TCC CTC GGT CGC TGA ACA AGA (SEQ ID NO:2); GAPDH: upper primer AAC TTT GTG AAG CTC ATT TCC TGG TAT (SEQ ID NO:3) und lower primer: CCT TGC TGG GCT GGG TGG T (SEQ ID NO:4))wurde mit der Primeranalyse-Software OLIGO®4.1 (National Biosciences, Plymoth, MN) so ausgesucht, dass die primerspezifische Schmelztemperatur zwischen 58 und 60°C lag. Die Länge der Fragmente betrug 116 bp (Katalase) und 123 bp (GAPDH). Die Analyse der Genexpression wurde dreimal wiederholt. 25 ng cDNA wurden mit Reaktionspuffer mit Tris-HCl, KCl und (NH₄)₂SO₄, 1.5 mM MgCl₂, 0.2 mM von jedem dNTP, 0.2 µM von jedem Primer, 0.1x SYBR Green I (Molecular Probes Europe, Leiden, Netherlands) und 1,2 U HotStart DNA Polymerase (Qiagen, Hilden) in einem Volumen von je 50 µl pro Ansatz einer Amplifikation unterzogen. Folgende PCR Parameter wurden verwendet: Initiierung bei 95 °C für 15 min., dann 40 Zyklen bei 95°C für 20 sec., 55 °C für 20 sec. und 72 °C für 20 sec. Um die Amplifikationspezifität zu prüfen wurden die PCR Produkte einer Schmelzpunktanalyse unterzogen. Die Quantifizierungsdaten wurden mit der iCycler iQ System Software (Bio-Rad Laboratories, Hercules, CA) berechnet: Nach Abzug der PCR Basislinie durch die Software wurde der lineare logarithmische Anteil der Fluoreszenz versus Zykluszahl aufgetragen, um die Zykluszahl (threshold cycle, C_{T}) zu bestimmen bei der die Schwellenwertfluoreszenz für Katalase und GAPDH als Referenz überschritten wurde. Da die Amplifikationseffektivität von Ziel- und Referenzgen annäherend gleich war (deltaC_{T} < 0.15), wurde die vergleichend C_{T} Methode zur Quantifizierung der Katalase im Verhältnis zu GAPDH verwendet.

Die Konzentration der mRNA für Katalase ohne Zinkinjektion war in Chorioidea-RPE-Komplexen von Albinos und pigmentierten Tieren genau gleich. Sechs Stunden nach Zinkinjektion war die mRNA für Katalase um den Faktor 12 erhöht. Nach 24 Stunden war dieser Unterschied verschwunden und Menge an mRNA für Katalase war wieder so hoch wie ohne Zinkinjektion. Diese Ergebnisse zeigern erstmals, dass Melanin die durch Zink induzierte Genexpression für Katalase, möglicherweise durch eine gesteigerte intrazelluläre Aufnahme, fördert.

### Beispiel 12: Melanin schützt vor Netzhautdegeneration, die durch Zinkmangel verursacht wurde.

Fünf Monate alte Albino (Wistar) Ratten und pigmentierte (Long Evans) Ratten wurden 42 Tage ausschließlich mit zinkarmer Diät (C1040, Altromin GmbH, Lage, Germany, Chargen Nr. 436) gefüttert. Die Augen wurden enukleiert und für die Elektronenmikroskopie nach Routinemethoden in Kunstoff eingebettet. An Semidünnschnitten wurden die noch vorhandenen Photorezeptoren in 4 Augen pro Gruppe ausgezählt. Pro Auge wurde die maximale Zahl der Photorezeptorreihen an 3 verschieden Stellen gezählt. Die Ergebnisse wurden mit Student's t-Test statistisch ausgewertet.

In beiden Versuchsgruppen war ein Photorezeptorverlust zu beobachten. Die Mittelwerte aus 12 Messungen der maximal vorhandenen Photorezeptorzellkeme betrug bei Albinoratten 5,3 ± 1 und bei pigmentierten Ratten 9,6 ± 0,6. Der Unterschied ist hoch signifikant (p = 0,00001). Die Photorezeptoraußensegmente waren bei pigmentierten Ratten etwa doppelt so lang wie bei Albinos. Diese Ergebnisse zeigen erstmals, dass Melanin vor Netzhautdegeneration, verursacht durch Zinkmangel, schützen kann. Wahrscheinlich konnte das in den Melanosomen gespeicherte Zink abgegeben werden, so dass die zinkabhängigen Zellfunktionen bei den pigmentierten Tieren besser und länger funktionierten als bei den Albinos.

### Beispiel 13: Zink in Kombination mit Melanin schützt Zellen vor letaler UVA Strahlung

30.000 humane amelanotische retinale Pigmentepithelzellen ARPE-19 (American Type culture Collection, Manassas, Via, USA) wurden in einer Mischung von DMEM and F12 Medium (PAA Laboratories, Linz, Austria) mit 10 % fötalem Kälberserum, Gentamycin und Amphotericin kultiviert und wurden mit lmg/ml Melanin von Tintenfischen (Sepia officinalis, Sigma) inkubiert. Nach 4 Stunden wurde das nicht phagozytierte Melanin gründlich ausgewaschen. Nach 24 Stunden wurden die künstlich pigmentierten Zellen für 2 Stunden mit 100 oder 200 µmol ZnCl₂ inkubiert. Drei Tage später, wurden die Zellen mit einer HRL, 125 W, Lampe (Radium, Germany) 1 Stunde lang bestrahlt. Der UVA Lichtanteil betrug an der Oberfläche des Zellkulturflüssigkeit 18 mW/cm² und wurde mit einem RM-12 UV Radiometer (Dr.Gröbel, UV Elektronik, Ettlingen, Germany) gemessen. Zellen ohne Melanin- und Zinkbehandlung mit und ohne Bestrahlung dienten der Kontrolle. Nach 2 weiteren Tagen wurde die Anzahl der überlebenden Zellen in einer Thomakammer ausgezählt.

In der Nachfolgenden Tabelle sind die Mittelwerte der überlebenden Zellen aus 3 unabhängigen ' Versuchen angegeben. Die Anzahl der überlebenden Zellen nach Bestrahlung ohne Melanin- und/oder Zinkbehandlung wurde mit der Anzahl der überlebenden Zellen mit Behandlung statistisch verglichen. Daher resultiert der p-Wert. Jede Behandlung hat die Überlebensrate der Zellen signifikant erhöht. Den besten Schutzeffekt hatte eine Kombination von Melaningabe und Inkubation mit 100 µmol Zink. Die Zahl der überlebenden Zellen war um den Faktor 7,4 im . Vergleich zu unbehandelten Zellen erhöht. Dies zeigt, dass eine Kombination aus intrazellulärer Melaninerhöhung und Zinksubstitution retinale Pigmentepithelzellen signifikant vor UVA-Phototoxizität schützt (siehe nachstehende Tabelle).

| Ohne Bestrahlung, ohne Zn, ohne Melanin | mit Bestrahlung, ohne Zn, ohne Melanin | mit Bestrahlung, mit. 100 µmol Zn | mit Bestrahlung, mit 200 µmol Zn | mit Bestrahlung; mit 100 µmol Zn, mit Melanin | mit Bestrahlung, ' mit 200 µmol Zn, mit Melamin |
|---|---|---|---|---|---|
| 335000±15000 | 11666±2886 | 33333+11547 | 41666±16072 | 86666±20207 | 70000±25000 |
| | | p = 0,03 | p = 0,03 | p =0,003 | p = 0,015 |

### Beispiel 14: Zink in Kombination mit Melanin vermindert die Bildung von H₂0₂ in retinalen Pigmentepithelzellen nach UVA Bestrahlung

30.000 humane amelanotische retinale Pigmentepithelzellen ARPE-19 (American Type culture Collection, Manassas, VA, USA) wurden in einer Mischung von DMEM and F12 Medium (PAA Laboratories, Linz, Austria) mit 10 % fötalem Kälberserum, Gentamycin' und Amphotericin kultiviert und wurden mit lmg/ml Melanin von Tintenfischen (Sepia officinalis, Sigma, Deisenhofen, Germany) inkubiert. Nach 4 Stunden wurde das nicht phagozytierte Melanin gründlich ausgewaschen. Nach 24 Stunden wurden die künstlich pigmentierten Zellen für 2 . Stunden mit 100 oder 200 µmol ZnCl₂ inkubiert. Die so behandelten und unbehandelten Zellen wurden mit 60 µM 2',7'-dichlorofluorescein Diacetate (Sigma, Deisenhofen, Germany), welches fluoresziert wenn es durch H₂0₂ oxidiert wird, inkubiert und 15 Minuten mit einer HRL, 125 W, ' Lampe (Radium, Germany) bestrahlt. Der UVA Lichtanteil betrug an der Oberfläche der Zellkulturflüssigkeit 18 mW/cm² und wurde mit einem RM-12 UV Radiometer (Dr.Gröbel, UV Elektronik, Ettlingen, Germany) gemessen. Zellen ohne Melanin- und Zinkbehandlung mit und ohne Bestrahlung dienten der Kontrolle. Die Fluoreszenzintensität wurde mit einem CytoFluor Multi-well Plate Reader (PerSeptive Biosystems, Wiesbaden Germany) gemessen.

In der Nachfolgenden Tabelle sind die Floureszenzintensitäten in Prozent angegeben, wobei geringere Floureszenz eine geringere Bildung von H₂O₂ anzeigt. Den besten Schutzeffekt hatte - eine Kombination von Melaningabe und Inkubation mit 100 µmol Zink. Die maximale Fluoreszenz in unbehandelten Zellen wurde gleich 100 % gesetzt. Die Fluoreszenzintensität wurde um 60 % im Vergleich zu unbehandelten Zellen reduziert (siehe nachfolgende Tabelle). Dies zeigt, dass eine Kombination aus intrazellulärer Melaninerhöhung und Zinksubstitution retinale Pigmentepithelzellen vor der Bildung von H₂0₂ induziert durch WA-Licht schützt (siehe nachstehende Tabelle).

| Fluoreszenzintensität mit Bestrahlung, ohne Zn, ohne Melanin | Fluoreszenzintensität mit Bestrahlung, mit Melanin und 100 µmol Zn | Fluoreszenzintensität mit Bestrahlung, mit Melanin ohne Zn |
|---|---|---|
| 100% | 40% | 79% |

### Referenzliste

1. C. K. Dorey, G. G. Khouri, L. A. Syniuta, S. A. Curran, J. J. Weiter, Invest Ophthalmol.Vis.Sci. 30, 1047-1054 (1989).
2. C..K..Dorey, X. Torres, T. Swart, Exp.Eye Res. 50, 1-10 (1990).
3. C. A. Ferguson and S. H. Kidson, Pigment Cell Res. 10, 127-138 (1997).
4. M.Miyamoto and T.B.Fitzpatrick, Science 126, 449-450 (1957).
5. T. Sarna, J.Photochem.Photobiol.B. 12, 215-258 (1992).
6. U. Schraermeyer and K. Heimann, Pigment Cell Res 12, 219-236 (1999).
7. Ebadi M, Leuschen MP, el RH, Hamada FM, Rojas P. Neurochem Int 29,159-166(1996).
8. Rimbach G, Markant A, PallaufJ, Kramer K. Z Ernahrungswiss 35, 123-142 (1996).
9. Frank RN, Amin RH, Puklin JE. Am J Ophthalmol 127, 694-709 (1999).
10. Barrenas ML, Lindgren F. Br J Audiol 25, 303-307 (1991).
11. Tate DJ, Jr., Miceli MV, Newsome DA. Free Radic Biol Med 26, 704-713 (1999).

## Patentansprüche

1. Verwendung von Tyrosinase, 5,6-Dihydroxyindol (DHI), 5,6-Dihydroxyindole-2-carboxylsäure (DHICA) und/oder 5-S-Cysteinyldopa (CD) in Kombination mit Zink zur Herstellung eines Arzneimittels zur Prävention oder Therapie von Erkrankungen des Auges, charakterisiert durch Drusenbildung oder einen erhöhten Gehalt an Lipofuszin.

2. Verwendung nach Anspruch 1, wobei Tyrosinase, 5,6-Dihydroxyindol (DHI), 5,6-Dihydroxyindole-2-carboxylsäure (DHICA) und/oder 5-S-Cysteinyldopa (CD) gleichzeitig mit Zink oder vor der Verabreichung von Zink zu verabreichen sind.

3. Tyrosinase, 5,6-Dihydroxyindol (DHI), 5,6-Dihydroxyindole-2-carboxylsäure (DHICA) und/oder 5-S-Cysteinyldopa (CD) in Kombination mit Zink zur Verwendung bei der Prävention oder Therapie von Erkrankungen des Auges, wobei die Erkrankungen des Auges durch Drusenbildung oder einen erhöhten Gehalt an Lipofuszin charakterisiert sind.

4. Tyrosinase,5,6-Dihydroxyindol (DHI), 5,6-Dihydroxyindole-2-carboxylsäure (DHICA) und/oder 5-S-Cysteinyldopa (CD) in Kombination mit Zink nach Anspruch 3, wobei Tyrosinase, 5,6-Dihydroxyindol (DHI), 5,6-Dihydroxyindole-2-carboxylsäure (DHICA) und/oder 5-S-Cysteinyldopa (CD) gleichzeitig mit Zink oder vor der Verabreichung von Zink zu verabreichen sind.

## Claims

1. Use of tyrosinase, 5,6-dihydroxyindole (DHI), 5,6-dihydroxyindole-2-carboxylic acid (DHICA) and/or 5-S-cysteinyldopa (CD) in combination with zinc for the preparation of a medicament for the prevention or therapy of diseases of the eye **characterized by** the formation of drusen or an increased content of lipofuscin.

2. Use according to claim 1, wherein tyrosinase, 5,6-dihydroxyindole (DHI), 5,6-dihydroxyindole-2-carboxylic acid (DHICA) and/or 5-S-cysteinyldopa (CD) have to be administered simultaneously with zinc or prior to the administration of zinc.

3. Tyrosinase, 5,6-dihydroxyindole (DHI), 5,6-dihydroxyindole-2-carboxylic acid (DHICA) and/or 5-S-cysteinyldopa (CD) in combination with zinc for use in the prevention or therapy of diseases of the eye, wherein the diseases of the eye are **characterized by** the formation of drusen or an increased content of lipofuscin.

4. Tyrosinase, 5,6-dihydroxyindole (DHI), 5,6-dihydroxyindole-2-carboxylic acid (DHICA) and/or 5-S-cysteinyldopa (CD) in combination with zinc according to claim 3, wherein tyrosinase, 5,6-dihydroxyindole (DHI), 5,6-dihydroxyindole-2-carboxylic acid (DHICA) and/or 5-S-cysteinyldopa (CD) have to be administered simultaneously with zinc or prior to the administration of zinc.

## Revendications

1. Utilisation d'une Tyrosinase, de 5,6-dihydroxyindole (DHI), d'acide 5,6-dihydroxyindole-2-carboxylique (DHICA) et/ou de 5-S-cystéinyldopa (CD) en combinaison avec du zinc pour la préparation d'un médicament destiné à la prévention ou à la thérapie des maladies de l'oeil **caractérisées par** la formation de druses ou une teneur élevée en lipofuscine.

2. Utilisation selon la revendication 1, dans laquelle la Tyrosinase, le 5,6-dihydroxyindole (DHI), l'acide 5,6-dihydroxyindole-2-carboxylique (DHICA) et/ou la 5-S-cystéinyldopa (CD) sont à administrer en même temps que le zinc ou avant l'administration du zinc.

3. Tyrosinase, 5,6-dihydroxyindole (DHI), acide 5,6-dihydroxyindole-2-carboxylique (DHICA) et/ou 5-S-cystéinyldopa (CD) en combinaison avec du zinc pour l'utilisation destiné à la prévention ou à la thérapie des maladies de l'oeil **caractérisées par** la formation de druses ou une teneur élevée en lipofuscine.

4. Tyrosinase, 5,6-dihydroxyindole (DHI), acide 5,6-dihydroxyindole-2-carboxylique (DHICA) et/ou 5-S-cystéinyldopa (CD) en combinaison avec du zinc selon la revendication 3, dans laquelle la Tyrosinase, le 5,6-dihydroxyindole (DHI), l'acide 5,6-dihydroxyindole-2-carboxylique (DHICA) et/ou la 5-S-cystéinyldopa (CD) sont à administrer en même temps que le zinc ou avant l'administration du zinc.


### SEQUENCE LISTING

<110> Schraermeyer, Ulrich
<120> Therapie von Erkrankungen des Auges, des Innenohres und des Zentralen Nervensystems
<130> CEV-002 PCT
<140> unknown
<141> 2003-02-12
<150> 10206723.6
<151> 2002-02-18
<160> 4
<170> PatentIn version 3.1
<210> 1
   <211> 25
   <212> DNA
   <213> Artificial Sequence
<220>
   <223> Primer
<400> 1
   tagccagaag agaaacccac aaact 25
<210> 2
   <211> 21
   <212> DNA
   <213> Artificial Sequence
<220>
   <223> Primer
<400> 2
   tccctcggtc gctgaacaag a 21
<210> 3
   <211> 27
   <212> DNA
   <213> Artificial Sequence
<220>
   <223> Primer
<400> 3
   aactttgtga agctcatttc ctggtat 27
<210> 4
   <211> 19
   <212> DNA
   <213> Artificial Sequence
<220>
   <223> Primer
<400> 4
   ccttgctggg ctgggtggt 19